# EUROPEAN PATENT APPLICATION

(11) **EP 4 818 299 A1**
(43) Date of publication of application: **07.10.2026**
(21) Application number: 25206333.4
(22) Date of filing: 02.10.2025
(51) Int. Cl.: C11D 3/20, A61Q 19/10, C11D 1/75, C11D 1/825, C11D 1/90, C11D 1/94, C11D 17/04, C11D 1/72, C11D 3/33, C11D 1/66, C11D 1/02

(54) **CLEANING PRODUCT**

(30) Priority: 02.04.2025 EP 25168083; 02.04.2025 EP 25168087
(71) Applicant: The Procter & Gamble Company, Cincinnati, OH 45202 (US)
(72) Inventor: BOUTOILLE, Alice Michele, 1853 Strombeek-Bever (BE); DE BIOURGE, Michael Bruno O., 1853 Strombeek-Bever (BE); DE MUNCK, Tom Roger Lisette, 1853 Strombeek-Bever (BE); GONZALES, Denis Alfred, 1853 Strombeek-Bever (BE); RENMANS, Marc Rene Bert, 1853 Strombeek-Bever (BE); SAVEYN, Pieter Jan Maria, 1853 Strombeek-Bever (BE)
(74) Representative: P&G Patent Belgium UK

(57) **Abstract**

The need for a cleaning product that combines a foam dispenser with a dishwashing composition that is capable of providing good crystalline grease cleaning while also providing good initial sudsing, is met by a cleaning product comprising a foam dispenser and a cleaning composition, the composition is housed in the foam dispenser and wherein the cleaning composition comprises an alkanediol in combination with an aminocarboxylate chelant.

## Description

### FIELD OF INVENTION

The present invention relates to a cleaning product comprising a foam dispenser and a cleaning composition.

### BACKGROUND OF THE INVENTION

Traditionally, manual dishwashing involves filling a sink with water, adding dishwashing detergent to create a soapy solution, immersing soiled dishes in the mixture, scrubbing the items, and rinsing to eliminate residual soils and suds. Historically, users washed an entire load of soiled dishware at once. However, with more and more people moving to smaller apartments, and having a greater desire to simplify dishwashing, users are increasingly preferring to clean individual items or small batches of dishware shortly after use. This shift often involves washing under running water instead of in a full sink, emphasizing the need for a quick and efficient cleaning process that minimizes user effort. In this context, users typically dispense a small quantity of detergent onto a sponge or directly onto the items to be cleaned.

To simplify the cleaning of small amounts of dishware, cleaning products featuring foam dispensers have emerged, allowing for more targeted application of smaller doses of cleaning compositions. Such dispensers combine the liquid dishwashing composition with air during dispensing, so that a foam is dispensed. Foam dispensers include spray dispensers whereby the liquid is dispensed under pressure as droplets that are mixed with the turbulent air during dispensing to provide the foam. Alternatively, the foam dispenser can comprise a mixing chamber in which the air and the liquid composition are combined to produce the foam. The latter dispenser typically comprises a mesh and/or orifice either in the mixing chamber or after the mixing chamber through which the liquid hand dishwashing composition and air are mixed to generate the foam.

Since the detergent composition is comprised in a foaming container, the composition can be uniformly applied to the surface of the article and left for a period in order to further remove grease residues. Especially during such uses, the user desires to see good foaming and foam endurance as this indicates sufficient cleaning efficacy remains. However, good grease removal remains challenging from detergent compositions applied using spray dispensers.

In particular, while foaming dishwashing products, such as dishwashing detergent spray products, offer convenience and ease of use, they face challenges when it comes to effectively removing crystalline fats. Crystalline fats, such as beef, lard, and other animal fats, can be particularly stubborn to remove since they are essentially solid and hard to dissolve. The interfacial tension and adhesion properties of crystalline fats make them resistant to regular dishwashing detergents, resulting in incomplete or unsatisfactory cleaning, or requiring greater manual effort.

To address this issue, high levels of hydrophobic solvents have been incorporated into dishwashing detergent sprays to enhance the removal of crystalline fats. Hydrophobic solvents, such as tripropylene glycol n-butyl ether and the like, are particularly effective for dissolving and dispersing fat molecules, making them more easily removable, even with little or no scrubbing. While such solvents are particularly effective at removing crystalline fats, they are challenging to stably formulate into detergent compositions at high enough levels, especially detergent compositions for hand dish spray applications, which have both low viscosity, and relatively low levels of surfactant. A less stable detergent spray composition may result in reduced performance, poor spray pattern, clogging of the nozzle, or limited shelf life, which may limit the commercial viability and consumer acceptance of such products.

Therefore, there is a need for an improved foaming dishwashing product that effectively removes crystalline fats without requiring excessive scrubbing, while also providing good sudsing.

WO2022199790A relates to a liquid detergent composition comprising or consisting of at least one hydroxyl compound selected from the group consisting of 1,2-hexanediol; 1,2-heptanediol; 1,2-octanediol; 1,2-decanediol; 2,3-heptanediol; 2,3-hexanediol; 2,3-octanediol; 2,3-nonanediol; glyceryl caprylate; 4-hydroxyacetophenone; and optionally tropolone or mixtures thereof. WO2023122098A discloses cleaning compositions, e.g., dishwashing compositions, comprising one or more anionic surfactants comprising sodium laureth sulfate (SLES), one or more amphoteric surfactants, and an additive selected from caprylyl glycol, ethanol, and a combination thereof. WO202489070A, WO202489067A, WO202489079A, WO202488521A, WO202488520A, WO2022199790A, US20230248626A, WO2022122143A, and US20210261887A relate to detergent compositions, including hand dishwashing detergent compositions, that comprise alkanediols.

EP25168083.1 relates to a cleaning product that combines a foam dispenser with a dishwashing composition that is capable of providing good cleaning while also providing both good initial sudsing and good enduring sudsing, the cleaning product comprising a foam dispenser and a cleaning composition, the composition is housed in the foam dispenser and wherein the cleaning composition comprises a high amount of amphoteric surfactant, zwitterionic surfactant, and mixtures thereof, in combination with a limited amount, or no, anionic surfactant, and an alkanediol comprising from 6 to 14 carbon atoms. EP25168087.2 relates to a cleaning product that combines a foam dispenser with a cleaning composition that is capable of providing good dish cleaning while also providing a copious volume of foam upon initial application, the cleaning product comprising a foam dispenser and a cleaning composition, the composition is housed in the foam dispenser and wherein the cleaning composition comprises a combination of an alkanediol which comprises alkyl chains having from 6 to 14 carbon atoms, and glycol ether solvent which is selected from the group consisting of: alkyl ethers of ethylene glycol or propylene glycol, and mixtures thereof.

EP3839025A relates to a cleaning product comprising a spray dispenser and a cleaning composition, which provides improved crystalline grease cleaning and good initial sudsing, and hence reduced time to clean the dishes, the cleaning product comprises a spray dispenser and a cleaning composition, the composition is housed in the spray dispenser and wherein the cleaning composition comprises alkyl polyglucoside surfactant, a co-surfactant selected from amphoteric surfactant, zwitterionic surfactant and mixtures thereof, and an organic solvent. WO2023143938A relates to a liquid dishwash composition for cleaning and disinfecting utensils, the compositions can be delivered as a spray from a handheld spray pump, the composition comprising a combination of two anionic surfactants, one non-ionic surfactant, a biodegradable chelating agent and a solvent. EP Application 23201537.0 relates to a liquid hand dishwashing detergent composition which provides for effective cleaning, as well as enhanced foaming and viscosity, the composition comprising an amine oxide amphoteric co-surfactant and mid-chain 1,2-alkanediol. EP Application 24179183.9 relates to a liquid hand dishwashing detergent composition comprising mid-chain 1,2-alkanediol and amine oxide amphoteric surfactant, which provides further improved foaming and viscosity. EP Application 23201583.4 relates to a liquid hand dishwashing detergent composition which provides for effective cleaning, as well as enhanced foaming, the composition comprising a surfactant system which comprises alkyl sulfated anionic surfactant having little or no alkoxylation, an amphoteric and/or zwitterionic co-surfactant, and nonionic surfactant, in combination with a mid-chain 1,2-alkanediol. WO95/10587A relates to a detergent composition for hard surfaces, the detergent composition providing good detergency and foaming while being useful for removal oily dirt in the kitchen. EP3118301B1 relates to a cleaning product, in particular, to a cleaning product comprising a spray dispenser and a cleaning composition for making the cleaning of dishware easier and faster. JP2016198765 relates to a high foaming cleaning method for tableware, especially for removing oil from portions of the dishware which are hard to reach or unreacheable by hand. WO2017204149A1 relates to a detergent composition which exhibits excellent detergency against solid fat-containing oil stains attached to hard surfaces, including tableware, wherein the detergent composition can be applied to the hard surface via a spray. WO2017204148A1 relates to a method for washing tableware without applying thereto mechanical force, by causing a liquid detergent composition which contains not less than 1 mass% of a surfactant, not less than 1 mass% of a chelating agent, and water, to be in contact with tableware having an oil stain such as a solid fat stain, wherein the mass ratio of the surfactant and chelant is not lower than 0.25 and the liquid detergent composition has an electrical conductivity at 25°C of not less than 0.70 S/m. JP2017210577A relates to a liquid detergent composition for tableware that has excellent low-temperature stability and can satisfactorily clean oil stains, including solid fat, attached to a surface of tableware, without rubbing with a flexible material such as sponge, and without applying mechanical force, by applying, for instance via a spray, a liquid detergent composition containing a branched anion surfactant, a glycol solvent having from 2 to 12 carbon atoms, and water. JP2017210576A relates to a liquid detergent composition for hard surfaces, including tableware, having excellent detergency on oil stains, including solid fat, attached to a plastic hard surface, and a method for cleaning a hard surface using the composition, the composition comprises a sulfosuccinic acid ester or a salt thereof, an anion surfactant containing a hydrocarbon group having carbon atoms of 8 or more and 21 or less and a sulfate ester group or a sulfonic acid group, a specific nonionic surfactant, and water. WO2017110773A relates to a liquid detergent composition for hand-dishwashing, including tableware, having excellent detergency on oil stains, the composition comprising a sulfosuccinic acid ester or a salt thereof, a further anionic surfactant having a hydrocarbon group with 8 to 21 carbon atoms and a sulfuric ester group or sulfonic acid group, an amphoteric surfactant, and water. WO2016110827A1 relates to a detergent solution which can be applied as a spray, for cleaning a receptacle for milk or liquid milk-derived products, the detergent solution comprising water, one or more types of surfactant and an odour absorbing compound, the surfactants dissolve greasy milk-based residues from the receptacle and the odour absorbing compound neutralises odours produced by any remaining milk-based residues not removed by the surfactants. WO2017011191A1 relates to a cleaning product comprising a spray dispenser and a cleaning composition housed in the spray dispenser, the composition comprises: 5% to 15% by weight of the composition of a surfactant system, wherein the surfactant composition comprises: ii. 40% to 90% by weight of the surfactant system of a non-ionic surfactant and 10 to 60% by weight of the surfactant system of a co-surfactant selected from anionic, amphoteric, zwitterionic and mixtures thereof; and a glycol ether solvent. US20070179079A and US20060009369A relate to a cleaning composition comprising a cationic biocide and adapted to clean a variety of hard surfaces, which can be applied via an impregnated material, or dispensed or sprayed as liquid from a container, or as a crystal, powder, paste, or otherwise semi-solid or solid form from a container. US20190055500A relates to an antimicrobial hard surface cleaning composition providing good antimicrobial efficacy, even at low levels of the antimicrobial agent, while also providing improved surface shine. US20100160201A relates to a cleaning composition with a limited number of natural ingredients which contains a hydrophobic syndetic, a hydrophilic syndetic, and a biguanide or a cationic quaternary ammonium salt, the cleaning composition can be used to clean laundry, soft surfaces, and hard surfaces. US20180002636A relates to a detergent solution for cleaning a receptacle for milk or liquid milk-derived products, the detergent solution comprising water, one or more types of surfactant and an odour absorbing compound, the surfactants are provided to dissolve greasy milk-based residues from the receptacle.

### SUMMARY OF THE INVENTION

The present invention relates to a cleaning product comprising a foaming dispenser and a cleaning composition, the composition is housed in the foam dispenser and wherein the cleaning composition comprises: from 2.0% to 25% by weight of the composition of a surfactant system; an alkanediol, wherein the alkyl chain of the alkanediol comprises from 6 to 14 carbon atoms; and an aminocarboxylate chelant..

### DETAILED DESCRIPTION OF THE INVENTION

Cleaning products comprising a foaming dispenser and a cleaning composition should provide excellent grease removal, including of hard to remove greases such as crystalline grease, while also providing good sudsing. By formulating the composition to comprise an alkanediol in combination with an aminocarboxylate chelant, as described herein, good removal of crystalline grease is achieved even with minimal scrubbing, while also providing a satisfactory sudsing experience.

The present invention relates to a cleaning product, which is a hand dishwashing cleaning product, the product comprising a foam dispenser and a cleaning composition. The cleaning composition is comprised within the foam dispenser.

For the purpose of the present invention "dishware" encompasses all the items used to either cook or used to serve and eat food.

By "spray dispenser" is herein meant a container comprising a housing to accommodate the composition and means to spray that composition. The preferred spraying means being a trigger spray. The composition of use in the present invention foams when it is sprayed on the surface to be treated.

As used in this specification and the claims that follow, the articles "a", "an", and "the" include singular and plural references unless the context clearly dictates otherwise. As such, the terms "a" or "an", "one or more" and "at least one" can be used interchangeably herein. Thus, for example, "a component" may include one or more component unless the reference is specifically indicated as being singular.

The comprising includes the terms consisting of and consisting essentially of.

All percentages are by weight of the total composition, as evident by the context, unless specifically stated otherwise. All ratios are weight ratios, unless specifically stated otherwise, and all measurements are made at 25°C, unless otherwise designated.

### Cleaning composition:

The cleaning composition is preferably a hand dishwashing cleaning composition, in liquid form. The cleaning composition is suitable for foaming.

Preferably the pH of the composition is greater than 7, preferably from 7 to 13, more preferably from 10 to 12 and most preferably from 10.5 to 11.5, as measured neat at 20°C. In the context of the compositions described herein, the aforementioned pH range has been found to provide both good cleaning, and grease-cleaning in particular.

Preferably, the composition has a reserve alkalinity of from 0.1 to 1, more preferably from 0.1 to 0.5 measured as described herein. This pH and the reserve alkalinity further contribute to the cleaning of tough food soils.

The cleaning product according to the invention can comprise a composition having a Newtonian viscosity, such as from 1 mPa.s to 50 mPa.s, preferably from 1 mPa.s to 35 mPa.s, more preferably from 1 mPa.s to 10 mPa.s, at 20°C as measured using the method defined herein.

Alternatively the cleaning product according to the invention can comprise a composition having a shear thinning rheology profile, such as having a high shear viscosity of from 1 mPa.s to 50 mPa.s, preferably from 1 mPa.s to 35 mPa.s, more preferably from 5 mPa.s to 15 mPa.s, when measured at a shear rate of at 1000 s⁻¹ at 20°C, and a low shear viscosity of from 100 mPa.s to 1,000 mPa.s, preferably from 200 mPa.s to 500 mPa.s, when measured at 0.1 s⁻¹ at 20°C, using the method defined herein.

Preferably the cleaning composition of use in the invention has a Newtonian viscosity.

The liquid cleaning composition typically comprises an aqueous carrier in which all the other composition actives are dissolved or eventually dispersed. As such, water can be present in an amount of from 60% to 90%, preferably from 75% to 85% by weight of the composition.

### Surfactant system:

The composition comprises from 2.0% to 25%, preferably from 5.0% to 20%, more preferably from 7.5% to 15% by weight of the composition of a surfactant system.

### First surfactant:

The surfactant system can comprise a first surfactant selected from the group consisting of: amphoteric surfactant, zwitterionic surfactant, and mixtures thereof.

The first surfactant can be present in the composition at a level of at least 25%, preferably from 40% to 90%, more preferably from 50% to 75% by weight of the surfactant system.

The cleaning composition can comprise from 2.0% to 15%, preferably from 5.0% to 12% by weight of the composition of the first surfactant. The cleaning composition can comprise a combination of amphoteric surfactant and zwitterionic surfactant, especially a combination of amine oxide amphoteric surfactant and betaine zwitterionic surfactant. When the cleaning composition comprises a combination of amphoteric surfactant and zwitterionic surfactant, the amphoteric surfactant and zwitterionic surfactant are preferably present in a weight ratio of from 1:1 to 10:1, preferably 2:1 to 5:1.

### Amphoteric surfactant:

The first surfactant can comprise or even consist of amphoteric surfactant. The amphoteric surfactant can comprise or even consist of amine oxide surfactant. Suitable amine oxide surfactants can be linear or branched, though linear are preferred. Suitable linear amine oxides are typically water-soluble, and characterized by the formula R1 - N(R2)(R3) O wherein R1 is a C8-18 alkyl, and the R2 and R3 moieties are selected from the group consisting of C1-3 alkyl groups, C1-3 hydroxyalkyl groups, and mixtures thereof. For instance, R2 and R3 can be selected from the group consisting of: methyl, ethyl, propyl, isopropyl, 2-hydroxethyl, 2-hydroxypropyl and 3-hydroxypropyl, and mixtures thereof, though methyl is preferred for one or both of R2 and R3. The linear amine oxide surfactants in particular may include linear C10-C18 alkyl dimethyl amine oxides and linear C8-C12 alkoxy ethyl dihydroxy ethyl amine oxides.

Preferably, the amine oxide surfactant is selected from the group consisting of: alkyl dimethyl amine oxide, alkyl amido propyl dimethyl amine oxide, and mixtures thereof. Alkyl dimethyl amine oxides are preferred, such as C8-18 alkyl dimethyl amine oxides, or C10-16 alkyl dimethyl amine oxides (such as coco dimethyl amine oxide). Suitable alkyl dimethyl amine oxides include C10 alkyl dimethyl amine oxide surfactant, C10-12 alkyl dimethyl amine oxide surfactant, C12-C14 alkyl dimethyl amine oxide surfactant, and mixtures thereof. C12-C14 alkyl dimethyl amine oxide are particularly preferred.

Alternative suitable amine oxide surfactants include mid-branched amine oxide surfactants. As used herein, "mid-branched" means that the amine oxide has one alkyl moiety having n1 carbon atoms with one alkyl branch on the alkyl moiety having n2 carbon atoms. The alkyl branch is located on the α carbon from the nitrogen on the alkyl moiety. This type of branching for the amine oxide is also known in the art as an internal amine oxide. The total sum of n1 and n2 can be from 10 to 24 carbon atoms, preferably from 12 to 20, and more preferably from 10 to 16. The number of carbon atoms for the one alkyl moiety (n1) is preferably the same or similar to the number of carbon atoms as the one alkyl branch (n2) such that the one alkyl moiety and the one alkyl branch are symmetric. As used herein "symmetric" means that | n1 - n2 | is less than or equal to 5, preferably 4, most preferably from 0 to 4 carbon atoms in at least 50 wt%, more preferably at least 75 wt% to 100 wt% of the mid-branched amine oxides for use herein. The amine oxide further comprises two moieties, independently selected from a C1-3 alkyl, a C1-3 hydroxyalkyl group, or a polyethylene oxide group containing an average of from about 1 to about 3 ethylene oxide groups. Preferably, the two moieties are selected from a C1-3 alkyl, more preferably both are selected as C1 alkyl.

Alternatively, the amine oxide surfactant can be a mixture of amine oxides comprising a mixture of low-cut amine oxide and mid-cut amine oxide. The amine oxide of the composition of the invention can then comprises:
a) from about 10% to about 45% by weight of the amine oxide of low-cut amine oxide of formula R1R2R3AO wherein R1 and R2 are independently selected from hydrogen, C1-C4 alkyls or mixtures thereof, and R3 is selected from C10 alkyls and mixtures thereof; and
b) from 55% to 90% by weight of the amine oxide of mid-cut amine oxide of formula R4R5R6AO wherein R4 and R5 are independently selected from hydrogen, C1-C4 alkyls or mixtures thereof, and R6 is selected from C12-C16 alkyls or mixtures thereof

In a preferred low-cut amine oxide for use herein R3 is n-decyl, with preferably both R1 and R2 being methyl. In the mid-cut amine oxide of formula R4R5R6AO, R4 and R5 are preferably both methyl.

Preferably, the amine oxide comprises less than about 5%, more preferably less than 3%, by weight of the amine oxide of an amine oxide of formula R7R8R9AO wherein R7 and R8 are selected from hydrogen, C1-C4 alkyls and mixtures thereof and wherein R9 is selected from C8 alkyls and mixtures thereof. Limiting the amount of amine oxides of formula R7R8R9AO improves both physical stability and suds mileage.

### Zwitterionic surfactant:

The first surfactant can comprise or even consist of zwitterionic surfactant. Zwitterionic surfactants have been found to improve the removal of polymerised or "baked-on" grease. Suitable zwitterionic surfactants include betaine surfactants. Such betaine surfactants includes alkyl betaines, alkylamidobetaine, amidazoliniumbetaine, sulphobetaine (INCI Sultaines) as well as the Phosphobetaine, and preferably meets formula (II):

R¹-[CO-X(CH₂)ₙ]ₓ-N⁺(R²)(R₃)-(CH₂)ₘ-[CH(OH)-CH₂]_{y}-Y-

wherein in formula (II),
R1 is selected from the group consisting of: a saturated or unsaturated C6-22 alkyl residue, preferably C8-18 alkyl residue, more preferably a saturated C10-16 alkyl residue, most preferably a saturated C12-14 alkyl residue;
X is selected from the group consisting of: NH, NR4 wherein R4 is a C1-4 alkyl residue, O, and S,
n is an integer from 1 to 10, preferably 2 to 5, more preferably 3,
x is 0 or 1, preferably 1,
R2 and R3 are independently selected from the group consisting of: a C1-4 alkyl residue, hydroxy substituted such as a hydroxyethyl, and mixtures thereof, preferably both R2 and R3 are methyl,
m is an integer from 1 to 4, preferably 1, 2 or 3,
y is 0 or 1, and
Y is selected from the group consisting of: COO, SO3, OPO(OR5)O or P(O)(ORS)O, wherein R5 is H or a C1-4 alkyl residue.

Preferred betaines are the alkyl betaines of formula (Ia), the alkyl amido propyl betaine of formula (Ib), the sulphobetaines of formula (Ic) and the amido sulphobetaine of formula (Id):

R¹-N(CH₃)₂-CH₂COO⁻ (IIa)

R¹-CO-NH-(CH₂)₃-N⁺(CH₃)₂-CH₂COO⁻ (IIb)

R¹-N⁺(CH₃)₂-CH₂CH(OH)CH₂SO₃⁻ (IIc)

R¹-CO-NH-(CH₂)₃-N⁺(CH₃)₂-CH₂CH(OH)CH₂SO₃⁻ (IId)

in which R1 has the same meaning as in formula (II). Particularly preferred are the carbobetaines [i.e. wherein Y-=COO- in formula (II)] of formulae (Ia) and (Ib), more preferred are the alkylamidobetaine of formula (Ib).

Suitable betaines can be selected from the group consisting or [designated in accordance with INCI]: capryl/capramidopropyl betaine, cetyl betaine, cetyl amidopropyl betaine, cocamidoethyl betaine, cocamidopropyl betaine, cocobetaines, decyl betaine, decyl amidopropyl betaine, hydrogenated tallow betaine / amidopropyl betaine, isostearamidopropyl betaine, lauramidopropyl betaine, lauryl betaine, myristyl amidopropyl betaine, myristyl betaine, oleamidopropyl betaine, oleyl betaine, palmamidopropyl betaine, palmitamidopropyl betaine, palm-kernelamidopropyl betaine, stearamidopropyl betaine, stearyl betaine, tallowamidopropyl betaine, tallow betaine, undecylenamidopropyl betaine, undecyl betaine, and mixtures thereof. Preferred betaines are selected from the group consisting of: cocamidopropyl betaine, cocobetaines, lauramidopropyl betaine, lauryl betaine, myristyl amidopropyl betaine, myristyl betaine, and mixtures thereof. Cocamidopropyl betaine is particularly preferred.

### Nonionic surfactant:

The surfactant system preferably further comprises nonionic surfactant, preferably wherein the surfactant system comprises the nonionic surfactant at a level of from 0.5% to 10%, more preferably from 1.0% to 7.5%, most preferably from 2.0% to 5.0% by weight of the composition. The nonionic surfactant can comprise or even consist of alkyl polyglucoside nonionic surfactant, alkoxylated preferably ethoxylated alcohol nonionic surfactant, and mixtures thereof. When the nonionic surfactant comprises both alkyl polyglucoside nonionic surfactant and alkoxylated alcohol nonionic surfactant, the alkyl polyglucoside nonionic surfactant and alkoxylated alcohol nonionic surfactant can be present in a weight ratio of from 4:1 to 1:4, preferably from 3:1 to 1:3, more preferably from 2:1 to 1:1 by weight of the composition.

### Alkyl polyglucoside surfactant:

The surfactant system preferably further comprises alkyl polyglucoside nonionic surfactant, preferably wherein the surfactant system comprises the alkyl polyglucoside nonionic surfactant at a level of from 0.5% to 7.5%, more preferably from 0.75% to 5.0%, most preferably from 1.0% to 3.5% by weight of the composition.

The alkyl polyglucoside nonionic surfactant can comprise or even consist of short-chain alkyl polyglucoside nonionic surfactant, wherein the short-chain alkyl polyglucoside nonionic surfactant has an average alkyl carbon chain length of from 8 carbon atoms to 10 carbon atoms and a number average degree of polymerization of from 0.5 to 3.0. The short-chain alkyl polyglucoside surfactant can have a number average degree of polymerization of from 0.1 to 3.0, preferably from 1.0 to 2.0, more preferably from 1.2 to 1.6. The weight ratio of C8 alkyl polyglucoside to C10 alkyl polyglucoside is preferably from 40:60 to 60:40. At least 90%, preferably at least 95%, more preferably at least 98%, by weight of the short-chain alkyl polyglucoside surfactant can have a carbon chain length of 8 to 10 carbon atoms.

Short-chain alkyl polyglucoside nonionic surfactants have also been found to improve grease cleaning performance.

Glucopon^{®} 215UP is a preferred C8-C10 alkyl polyglucoside surfactant. Other suitable short-chain alkyl polyglucosides include PLANTACARE^{®} 810 UP, Supplied by BASF.

For improved initial sudsing, the alkyl polyglucoside surfactant can have a number average alkyl carbon chain length between 8 and 18, preferably between 8 and 14, most preferably between 8 and 10, with an average degree of polymerization of between 0.1 and 3.0 preferably between 1.0 and 2.0, most preferably between 1.2 and 1.6.

Alkyl polyglucoside nonionic surfactants are typically more sudsing than other nonionic surfactants such as alkyl ethoxlated alcohols, especially in the presence of particulate soils.

C8-C18 alkyl polyglucosides are commercially available from several suppliers (e.g., Simusol^{®} surfactants from Seppic Corporation; and Glucopon^{®} 600 CSUP, Glucopon^{®} 650 EC, Glucopon^{®} 600 CSUP/MB, and Glucopon^{®} 650 EC/MB, from BASF Corporation).

### Alkoxylated nonionic surfactant:

The surfactant system can comprise alkoxylated nonionic surfactant. If present, the surfactant system can comprise from 0.5% to 10%, preferably from 0.75% to 5.0%, more preferably from 1.0% to 3.0% by weight of the composition of the alkoxylated nonionic surfactant.

Suitable alkoxylated nonionic surfactants include alkyl alkoxylated nonionic surfactants, more preferably alkyl ethoxylated nonionic surfactants. The alkyl chain can either be straight or branched, primary or secondary, preferably straight.

The further nonionic surfactant is preferably a low-cut alkyl ethoxylate nonionic surfactant. Low-cut alcohol ethoxylate nonionic surfactants include alcohol ethoxylate nonionic surfactants with an average alkyl carbon chain length of C10 and below. More preferably the alkyl ethoxylate surfactant has an average alkyl chain length of between C5 to C8, preferably between C5 to C7, and a number average degree of ethoxylation of from 1.0 to 10, preferably from 3.0 to 8.0, more preferably from 4.0 to 6.0. Suitable alcohol ethoxylate nonionic surfactants include commercially available materials such as Emulan^{®} HE50 or Lutensol^{®} CS6250 (available from BASF).

Alternatively, or in addition, the alkyl alkoxylated nonionic surfactant can comprise an alkoxylated alcohol nonionic surfactant which is a linear or branched, primary or secondary alkyl alkoxylated nonionic surfactant, preferably an alkyl ethoxylated nonionic surfactant, comprising on average from 10 to 14 carbon atoms in its alkyl chain and on average from 5 to 12, preferably from 6 to 10, most preferably from 7 to 8, units of ethylene oxide per mole of alcohol. The alkyl chain is preferably essentially linear. Suitable examples of commercially available longer chain alkoxylated alcohol nonionic surfactants include, those derived from alcohols sold under the Neodol^{®} brand-name by Shell, or the Lial^{®}, Isalchem^{®}, and Safol^{®} brand-names by Sasol, or some of the natural alcohols produced by The Procter & Gamble Chemicals company. The performance can be affected by the width of the alkoxylation distribution of the alkoxylated alcohol nonionic surfactant. The alkoxylation distribution, including its broadness can be varied through the selection of catalyst and process conditions when making the alkoxylated alcohol nonionic surfactant.

Other suitable nonionic surfactants for use herein can be selected from fatty alcohol polyglycol ethers, fatty acid glucamides, and mixtures thereof.

### Other surfactant:

The compositions of use in the present invention can comprise relatively low amounts of anionic surfactant. If present, anionic surfactant is present at a level of less than 25% by weight of the surfactant system of the anionic surfactant. The anionic surfactant is preferably present at a level of less than 15%, more preferably less than 10%, most preferably less than 5% by weight of the surfactant system. More preferably, the surfactant system is essentially free, or even free of anionic surfactant.

Should the detergent composition comprise an anionic surfactant, suitable anionic surfactants include, but are not limited to, those surface-active compounds that contain an organic hydrophobic group containing generally 8 to 22 carbon atoms or generally 8 to 18 carbon atoms in their molecular structure and at least one water-solubilizing group preferably selected from sulfonate, sulfate, and carboxylate so as to form a water-soluble compound. Usually, the hydrophobic group will comprise a linear or branched C8-C22 alkyl, or acyl group. Such surfactants are employed in the form of water-soluble salts and the salt-forming cation usually is selected from sodium, potassium, ammonium, magnesium and mono-, di- or tri-alkanolammonium, with the sodium, cation being the usual one chosen.

Fatty acids typically impede formation of suds in the presence of wash water comprising water hardness. However, for sprays and foams, fatty acids have been found to be able to improve initial suds formation while also improve the rinsing away of suds, especially where the water used for rinsing comprises water hardness. The surfactant system can comprise from 0.1% to 2.0%, preferably from 0.15% to 1.0%, more preferably from 0.25% to 0.75%, by weight of the composition of fatty acid or salt thereof. The fatty acid can be neutralised. Suitable fatty acids include palm kernel fatty acid, coconut fatty acid, rapeseed fatty acid, and salts thereof, with palm kernel fatty acid and salts thereof being particularly preferred. Where the fatty acid is partially or fully in its salt form, the fatty acid can have been neutralised with an (earth) alkali metal, an amine, or a mixture thereof. Suitable amines include: monoethanolamine, triethanolamine or mixtures thereof. Suitable alkali (earth) metals include: sodium, potassium, magnesium or a mixture thereof. The fatty acid may be branched or linear but since it is derived from natural feedstocks, is typically linear. Though not preferred, the fatty acid can be alkoxylated and/or hydroxylated.

The compositions of use in the present invention are preferably free of cationic surfactant and especially free of antimicrobial cationic surfactants, since such surfactants are typically detrimental to grease cleaning and surface shine. Such antimicrobial cationic surfactants include quaternary ammonium compounds such as dodecyl dimethyl ammonium chloride, alkyl dimethyl benzyl ammonium chloride, alkyl dimethyl ethylbenzyl ammonium chloride, and mixtures thereof.

### Alkanediol:

The cleaning composition comprises from 0.1% to 10% by weight of the composition of the alkanediol. Preferably, the cleaning composition comprises the alkanediol at a level of from 0.25% to 4.0%, preferably from 0.25% to 3.0%, more preferably from 0.5% to 2.0% by weight of the composition.

The alkyl chain of the alkanediols of use in the present invention comprises from 6 to 14, more preferably from 8 to 12, most preferably from 8 to 10 carbon atoms. The alkanediol is preferably a 1,2-alkanediol. The alkyl chain of the 1,2-alkanediol is preferably a linear alkyl chain.

Suitable 1,2-alkanediols include: 1,2-hexanediol, 1,2-heptanediol, 1,2-octanediol, 1,2-nonanediol, 1,2-decanediol, 1,2-undecanediol, 1,2-dodecanediol, 1,2-tridecanediol, 1,2-tetradecanediol, or mixtures thereof.

Preferably the 1,2-alkanediol comprises an even number of carbon atoms, and more preferably the alkyl chain is derived from natural sources, such as fatty acids. Suitable C6 to C14 1,2-alkanediols include straight alkyl chain 1,2-hexanediol, 1,2-octanediol, 1,2-decanediol, 1,2-dodecanediol, 1,2-tetradecanediol, or mixtures thereof, preferably 1,2-octanediol, 1,2-decanediol, 1,2-dodecanediol, and mixtures thereof, most preferably 1,2-octanediol, 1,2-decanediol, and mixtures thereof.

At least 50%, preferably at least 80%, more preferably at least 98% by weight of the 1,2-alkanediols present in the composition are C8 to C12 1,2-alkanediols, or C8 to C10 1,2-alkanediols, with 1,2-octanediol being particularly preferred. Compositions comprising such 1,2-alkanediols have been shown to provide improved phase stability.

At least 50%, preferably at least 80%, more preferably at least 98% by weight of the 1,2-alkanediols present in the composition can be alkanediol, comprising from 6 to 14 carbon atoms. Even more preferably, the liquid composition is essentially free or even free of any 1,2-alkanediol comprising alkyl chains having less than 6 or more than 14 carbon atoms.

The cleaning composition can comprise the alkanediol and the surfactant system in a weight ratio of from 1:20 to 1:1, preferably from 1:15 to 1:2, more preferably 1:10 to 1:3.

Suitable 1,2-alkanediols products are available from the Symrise or the Sigma Aldrich companies.

Alkanediols and compositions comprising them are further described in US2025/0207062 (Symrise), US20250115831, US20250115829, and US20250115830.

### Aminocarboxylate chelant:

The composition herein comprises an aminocarboxylate. The aminocarboxylate can be present at a level of from 0.1% to 10%, preferably from 0.2% to 5%, more preferably from 0.2% to 3%, most preferably from 0.5% to 1.5% by weight of the composition.

The aminocarboxylate chelants can be selected from: methyl glycine diacetate (MGDA), glutamic acid N, N-diacetic acid (GLDA), ethylenediamine-N,N'-disuccinic acid (EDDS), and mixtures thereof, preferably from methyl glycine diacetate (MGDA), glutamic acid N, N-diacetic acid (GLDA), and mixtures thereof, more preferably glutamic acid N, N-diacetic acid (GLDA).

Glutamic Acid Diacetic acid (GLDA) may be added to the composition in acid form, as a salt, or as a diacetate.

Glutamic Acid Diacetic acid (GLDA) may be present in the composition as an acid or salt form, depending on the composition pH, or a mixture of acid and salt. Preferred salt forms include mono-, di-, tri- or tetraalkali metal and mono-, di-, tri- or tetraammonium salts of GLDA. Alkali metal salts of glutamic acid diacetic acid GDLA are preferably selected from lithium salts, potassium salts and more preferably sodium salts of GLDA, particularly the tetrasodium salt (Tetrasodium Glutamate Diacetate).

Glutamic acid diacetic acid can be partially or preferably fully neutralized with the respective alkali. Preferably, an average of from 3.5 to 4 COOH groups of GLDA is neutralized with alkali metal, preferably with sodium. Most preferably the composition comprises a tetrasodium salt of GLDA. GLDA can be at least partially neutralized with alkali metal, more preferably with sodium or potassium, most preferred with sodium.

GLDA is preferably present as the L-enatiomer (L-glutamic acid N, N-diacetic acid) or a racemic blend of the L- and D- enantiomers, since the L-enatiomer is more readily biodegradeable. The GLDA salt may be an alkali metal salt of L-GLDA, an alkali metal salt of D-GLDA, or enantiomerically enriched mixtures of isomers. Preferably the composition comprises a mixture of L- and D- enantiomers of glutamic acid diacetic acid (GLDA) or its respective mono-, di-, tri-, or tetraalkali metal or mono-, di-, trior tetraammonium salt or mixtures thereof, said mixtures containing predominantly the respective L-isomer with an enantiomeric excess in the range of from 10 to 95%. Preferably the GLDA salt is essentially L-glutamic acid diacetic acid that is at least partially neutralized with alkali metal.

Sodium salts of GLDA are preferred.

A suitable commercial source of GLDA in the form of the tetrasodium salt is DISSOLVINE^{®} GL, available from Nouryon.

Preferred salt forms include mono-, di-, tri- or tetraalkali metal and mono-, di-, tri- or tetraammonium salts of MGDA. Alkali metal salts are preferably selected from sodium salts, lithium salts, potassium salts, with sodium salts of MGDA being preferred. Especially preferred is the trisodium salt of MGDA.

MGDA can be partially or preferably fully neutralized with the respective alkali metal. Preferably, an average of from 2.7 to 3 COOH groups per molecule of MGDA is neutralized with alkali metal, preferably with sodium.

MGDA can be selected from racemic mixtures of alkali metal salts of MGDA and of the pure enantiomers such as alkali metal salts of L-MGDA, alkali metal salts of D-MGDA and of mixtures of enantiomerically enriched isomers.

Suitable commercial sources of MGDA in the form of the trisodium salt are TRILON^{®} M available from BASF and Dissolvine^{®} M-40 from Nouryon.

Ethylenediamine-N,N'-disuccinic acid (EDDS) is an aminopolycarboxylic acid which has two chiral centers, and as such three stereoisomers. These are the enantiomeric (R,R) and (S,S) isomers and the achiral meso (R,S) isomer. For improved biodegradability, the (S,S) stereoisomer is particularly preferred.

Ethylenediamine-N,N'-disuccinic acid (EDDS) may be present in acid or salt form, depending on the composition pH, or a mixture of acid and salt. Preferred salt forms include mono-, di-, tri- or tetraalkali metal and mono-, di-, tri- or tetraammonium salts of EDDS. Alkali metal salts of EDDS are preferably selected from lithium salts, potassium salts and more preferably sodium salts. Ethylenediamine-N,N'-disuccinic acid (EDDS) may be partially or preferably fully neutralized with the respective alkali.

Minor amounts of the aminocarboxylate chelants may bear a cation other than alkali metal. It is thus possible that minor amounts, such as 0.01 to 5 mol-% bear alkali earth metal cations such as Mg2+ or Ca2+, or an Fe(II) or Fe(III) cation. GLDA may contain minor amounts of impurities stemming from its synthesis, such as lactic acid, alanine, propionic acid or the like. "Minor amounts" in this context refer to a total of 0.1 to 1% by weight, referring to sequestering agent aminocarboxylate.

The cleaning composition can comprise the aminocarboxylate chelant and the surfactant system in a weight ratio of from 1:25 to 1:1, preferably from 1:20 to 1:3, more preferably 1:15 to 1:5.

The aminocarboxylate chelants and the alkanediol can be present in a weight ratio of from 4:1 to 1:4, preferably from 3:1 to 1:3, most preferably from 2:1 to 1:2.

### Glycol ether solvent:

The composition preferably further comprises glycol ether solvent. Suitable glycol ether solvents can be selected from the group consisting of: alkyl or aryl ethers of ethylene glycol or propylene glycol, and mixtures thereof, with alkyl ethers being preferred. Such glycol ether solvents have been found to further improve foam volume, especially when used in combination with alkanediols of use in the invention, and/or with alkoxylated nonionic surfactants.

The alkyl groups are formed by the removal of one hydrogen atom from an alkane chain, converting it from -ane to -yl. In contrast, aryl groups are formed by the removal of hydrogen from the aromatic ring.

Such glycol ether solvents also improve penetration and removal of crystalline grease and are particularly effective when used in combination with the alkyl polyglucoside to remove crystalline grease, and can also improve sudsing.

The composition can comprise from 0.1% to 10%, preferably from 0.5% to 8.0%, more preferably from 1.0% to 5.0% by weight of the total composition of the glycol ether solvent.

The glycol ether solvent is selected from the group consisting of: alkyl or aryl ethers of ethylene glycol or propylene glycol, and mixtures thereof. Suitable such glycol ether solvents can be selected from:
a) Formula (I): R¹O(R²O)ₘR³, wherein R¹ is a linear or branched C4, C5 or C6 alkyl or a substituted or unsubstituted phenyl; R² is ethyl or isopropyl; R³ is hydrogen or methyl; and m is 1, 2 or 3, preferably 1 or 2;
b) Formula (II): R⁴O(R⁵O)ₙR⁶, wherein R⁴ is n-propyl or isopropyl; R⁵ is isopropyl; R⁶ is hydrogen or methyl; and n is 1, 2 or 3, preferably 1 or 2;
c) Formula (III): R⁷O(R⁸O)ₚR⁹, wherein R⁷ is methyl or ethyl; R⁸ is ethyl or isopropyl; R⁹ is hydrogen or methyl, preferably hydrogen; and p is 1, 2 or 3, preferably 1 or 2;
d) Formula (IV): R¹⁰O(R¹¹O)_{q}R¹², wherein R¹⁰ is n-propyl or isopropyl, preferably n-propyl; R¹¹ is ethyl; R¹² is hydrogen or methyl, preferably hydrogen; and q is 1, 2 or 3, preferably 1 or 2; and
e) mixtures thereof, preferably Formula (I), Formula (II), and mixtures thereof, preferably Formula (I) and mixtures thereof.

Suitable glycol ether solvents according to Formula I include: ethyleneglycol n-butyl ether, diethyleneglycol n-butyl ether, triethyleneglycol n-butyl ether, propyleneglycol n-butyl ether, dipropyleneglycol n-butyl ether, tripropyleneglycol n-butyl ether, ethyleneglycol n-pentyl ether, diethyleneglycol n-pentyl ether, triethyleneglycol n-pentyl ether, propyleneglycol n-pentyl ether, dipropyleneglycol n-pentyl ether, tripropyleneglycol n-pentyl ether, ethyleneglycol n-hexyl ether, diethyleneglycol n-hexyl ether, triethyleneglycol n-hexyl ether, propyleneglycol n-hexyl ether, dipropyleneglycol n-hexyl ether, tripropyleneglycol n-hexyl ether, ethyleneglycol isobutyl ether, diethyleneglycol isobutyl ether, triethyleneglycol isobutyl ether, propyleneglycol isobutyl ether, dipropyleneglycol isobutyl ether, tripropyleneglycol isobutyl ether, ethyleneglycol isopentyl ether, diethyleneglycol isopentyl ether, triethyleneglycol isopentyl ether, propyleneglycol isopentyl ether, dipropyleneglycol isopentyl ether, tripropyleneglycol isopentyl ether, ethyleneglycol isohexyl ether, diethyleneglycol isohexyl ether, triethyleneglycol isohexyl ether, propyleneglycol isohexyl ether, dipropyleneglycol isohexyl ether, tripropyleneglycol isohexyl ether, ethyleneglycol phenyl ether, diethyleneglycol phenyl ether, triethyleneglycol phenyl ether, propyleneglycol phenyl ether, dipropyleneglycol phenyl ether, tripropyleneglycol phenyl ether, ethyleneglycol n-butyl methyl ether, diethyleneglycol n-butyl methyl ether triethyleneglycol n-butyl methyl ether, propyleneglycol n-butyl methyl ether, dipropyleneglycol n-butyl methyl ether, tripropyleneglycol n-butyl methyl ether, ethyleneglycol n-pentyl methyl ether, diethyleneglycol n-pentyl methyl ether, triethyleneglycol n-pentyl methyl ether, propyleneglycol n-pentyl methyl ether, dipropyleneglycol n-pentyl methyl ether, tripropyleneglycol n-pentyl methyl ether, ethyleneglycol n-hexyl methyl ether, diethyleneglycol n-hexyl methyl ether, triethyleneglycol n-hexyl methyl ether, propyleneglycol n-hexyl methyl ether, dipropyleneglycol n-hexyl methyl ether, tripropyleneglycol n-hexyl methyl ether, ethyleneglycol isobutyl methyl ether, diethyleneglycol isobutyl methyl ether, triethyleneglycol isobutyl methyl ether, propyleneglycol isobutyl methyl ether, dipropyleneglycol isobutyl methyl ether, tripropyleneglycol isobutyl methyl ether, ethyleneglycol isopentyl methyl ether, diethyleneglycol isopentyl methyl ether, triethyleneglycol isopentyl methyl ether, propyleneglycol isopentyl methyl ether, dipropyleneglycol isopentyl methyl ether, tripropyleneglycol isopentyl methyl ether, ethyleneglycol isohexyl methyl ether, diethyleneglycol isohexyl methyl ether, triethyleneglycol isohexyl methyl ether, propyleneglycol isohexyl methyl ether, dipropyleneglycol isohexyl methyl ether, tripropyleneglycol isohexyl methyl ether, ethyleneglycol phenyl methyl ether, diethyleneglycol phenyl methyl ether, triethyleneglycol phenyl methyl ether, propyleneglycol phenyl methyl ether, dipropyleneglycol phenyl methyl ether, tripropyleneglycol phenyl methyl ether, and mixtures thereof.

Preferred glycol ether solvents according to Formula I are: ethyleneglycol n-butyl ether, diethyleneglycol n-butyl ether, triethyleneglycol n-butyl ether, propyleneglycol n-butyl ether, dipropyleneglycol n-butyl ether, tripropyleneglycol n-butyl ether, ethyleneglycol n-hexyl ether, ethylene glycol phenyl ether and mixtures thereof, more preferably propyleneglycol n-butyl ether, dipropyleneglycol n-butyl ether, tripropyleneglycol n-butyl ether, ethyleneglycol n-hexyl ether, and mixtures thereof.

The most preferred glycol ether solvents according to Formula I are: dipropyleneglycol n-butyl ether, ethyleneglycol n-hexyl ether, and mixtures thereof.

Suitable glycol ether solvents according to Formula II include: propyleneglycol n-propyl ether, dipropyleneglycol n-propyl ether, tripropyleneglycol n-propyl ether, propyleneglycol isopropyl ether, dipropyleneglycol isopropyl ether, tripropyleneglycol isopropyl ether, propyleneglycol n-propyl methyl ether, dipropyleneglycol n-propyl methyl ether, tripropyleneglycol n-propyl methyl ether, propyleneglycol isopropyl methyl ether, dipropyleneglycol isopropyl methyl ether, tripropyleneglycol isopropyl methyl ether, and mixtures thereof.

Preferred glycol ether solvents according to Formula II are: propyleneglycol n-propyl ether, dipropyleneglycol n-propyl ether, tripropyleneglycol n-propyl ether and mixtures thereof.

Suitable glycol ether solvents according to Formula III include: ethyleneglycol methyl ether, diethyleneglycol methyl ether, triethyleneglycol methyl ether, ethyleneglycol ethyl ether, diethyleneglycol ethyl ether, triethyleneglycol ethyl ether, ethyleneglycol methyl methyl ether, diethyleneglycol methyl methyl ether, triethyleneglycol methyl methyl ether, ethyleneglycol ethyl methyl ether, diethyleneglycol ethyl methyl ether, triethyleneglycol ethyl methyl ether, propyleneglycol methyl ether, dipropyleneglycol methyl ether, tripropyleneglycol methyl ether, propyleneglycol ethyl ether, dipropyleneglycol ethyl ether, tripropyleneglycol ethyl ether, propyleneglycol methyl methyl ether, dipropyleneglycol methyl methyl ether, tripropyleneglycol methyl methyl ether, propyleneglycol ethyl methyl ether, dipropyleneglycol ethyl methyl ether, tripropyleneglycol ethyl methyl ether, and mixtures thereof.

Preferred glycol ether solvents according to Formula III are: propyleneglycol methyl ether, dipropyleneglycol methyl ether, tripropyleneglycol methyl ether, and mixtures thereof.

The most preferred glycol ether solvent according to Formula III is tripropyleneglycol methyl ether.

Suitable glycol ether solvents according to Formula IV include: ethyleneglycol n-propyl ether, diethyleneglycol n-propyl ether, triethyleneglycol n-propyl ether, ethyleneglycol isopropyl ether, diethyleneglycol isopropyl ether, triethyleneglycol isopropyl ether, ethyleneglycol n-propyl methyl ether, diethyleneglycol n-propyl methyl ether, triethyleneglycol n-propyl methyl ether, ethyleneglycol isopropyl methyl ether, diethyleneglycol isopropyl methyl ether, triethyleneglycol isopropyl methyl ether, and mixtures thereof.

Preferred glycol ether solvents according to Formula IV are: ethyleneglycol n-propyl ether, diethyleneglycol n-propyl ether, triethyleneglycol n-propyl ether and mixtures thereof.

The most preferred glycol ether solvents are dipropyleneglycol n-butyl ether, ethyleneglycol n-hexyl ether, tripropyleneglycol methyl ether, and mixtures thereof, especially dipropyleneglycol n-butyl ether.

Suitable glycol ether solvents can be purchased from The Dow Chemical Company, in particularly from the E-series (ethylene glycol based) Glycol Ether and the P-series (propylene glycol based) Glycol Ether line-ups. Suitable glycol ether solvents include Butyl Carbitol, Hexyl Carbitol, Butyl Cellosolve, Hexyl Cellosolve, Butoxytriglycol, Dowanol PnP, Dowanol DPnP, Dowanol PnB, Dowanol DPnB, Dowanol TPnB, Dowanol EPh and mixtures thereof.

The surfactant system and the glycol ether solvent are preferably in a weight ratio of from 10:1 to 1:2, preferably from 8:1 to 1:1, most preferably 7:1 to 3:1. Compositions of use in the present invention, having such a weight ratio of surfactant system to glycol ether solvent have been found to provide improved coverage on the dishware with minimum over-spray (residual spray droplets remaining in suspension in the air). Therefore, such spray compositions reduce wastage and minimise the amount of spray droplets which can be inhaled. Compositions having a surfactant:glycol ether solvent weight ratio lower than 1:2 have been found to be less foaming and/or have a greater tendency to phase separate over time. Compositions having a surfactant: glycol ether solvent weight ratio higher than 10:1 are typically more difficult to spray and are more prone to gelling when sprayed onto greasy soils, when the soil is not first wetted. Such gel formation inhibits the spreading of the composition onto the greasy surface and hence leads to less satisfactory cleaning.

Glycol ether solvents are further described in US2017/0015946, US10689596, US10138445, US10150937, US10138446, US10934509, US2018/0371362, US2018/0371370, and US11180715.

### Further solvents:

Suitable further organic solvents can be selected from the group consisting of: alcohol solvents, ester solvents, and mixtures thereof. Suitable alcohols can be selected from the group consisting of C4-C6 linear mono-alcohols, branched C4-C10 mono-alcohols having one or more C1-C4 branching groups, alkyl mono-glycerols, and mixtures thereof.

Preferred C4-C6 linear mono-alcohols are selected from pentanol, hexanol, and mixtures thereof, preferably 1-pentanol, 1-hexanol, and mixtures thereof.

Preferred branched C4-C10 mono-alcohols having one or more C1-C4 branching groups for use herein are C4-C8 primary mono-alcohols having one or more C1-C4 branching groups, and mixtures thereof. Especially preferred branched C4-C10 mono-alcohols having one or more C1-C4 branching groups for use herein include methyl butanol, ethyl butanol, methyl pentanol, ethyl pentanol, methyl hexanol, ethyl hexanol, propyl hexanol, dimethyl hexanol trimethyl hexanol, methyl hepanol, ethyl heptanol, propyl heptanol, dimethyl heptanol,trimethyl heptanol, methyl octanol, ethyl octanol, propyl octanol, butyl octanol, dimethyl octanol, trimethyl octanol, methyl nonanol, ethyl nonanol, propyl nonanol, butyl nonanol, dimethyl nonanol and trimethyl nonanol, and mixtures thereof. More preferred for use herein are the primary 1-alcohol member of branched C4-C10 mono-alcohols having one or more C1-C4 branching groups, especially preferred are the primary 1-alcohol family members of methyl butanol, ethyl butanol, methyl pentanol, ethyl pentanol, methyl hexanol, ethyl hexanol, propyl hexanol, dimethyl hexanol trimethyl hexanol, methyl hepanol, ethyl heptanol, propyl heptanol, dimethyl heptanol,trimethyl heptanol, methyl octanol, ethyl octanol, propyl octanol, butyl octanol, dimethyl octanol, trimethyl octanol, methyl nonanol, ethyl nonanol, propyl nonanol, butyl nonanol, dimethyl nonanol, trimethyl nonanol, and mixtures thereof.

More preferred alcohols are butyl octanol, trimethyl hexanol, ethyl hexanol, propyl heptanol, methyl butanol, and mixtures thereof, in particular the primary 1-alcohol family member, more in particular ethyl hexanol, butyl octanol, trimethyl hexanol, and mixtures thereof, especially 2-ethyl-1-hexanol, 2-butyl-1-octanol, 3,5,5 trimethyl-1-hexanol, and mixtures thereof.

Preferred alkyl mono-glycerols are selected from the group consisting of branched alkyl mono-glycerols and mixtures thereof, more preferably branched C4-C8 alkyl mono-glycerols with one or more C1 to C4 alkyl branching groups, more preferably selected from the group consisting of ethylhexylglycerol, propylheptylglycerol, and mixtures thereof, most preferably 2-ethylhexylglycerol.

Such alcohols can also improve sudsing.

Especially preferred for use herein are mixtures of mono-alcohols, in particular mixtures comprising a branched C4-C10 mono-alcohol, more in particular mixtures comprising an alcohol selected from the group comprising C4-C8 more preferably C6-C7 branched primary alcohols. Preferably for use is a mixture of alcohols comprising an alcohol selected from the group comprising C4-C8 branched primary alcohols with an alcohol selected of the group of C4-C6 linear mono-alcohols and alkylglycerols. Such mixtures can boost foaming and improve cleaning of various oily soils.

Suitable ester solvents can be selected from the group consisting of monoester solvents of Formula III, di- or triester solvents of formula IV, benzylbenzoate, and mixtures thereof.
a) Monoester solvents of formula III: R1C=OOR2, wherein:
   R1 is a linear of branched C1 to C4 alkyl, preferably a linear or branched C2 to C3 alkyl;
   R2 is a linear or branched C2 to C8 alkyl, preferably a linear or branched C2 to C6 alkyl, most preferably a linear of branched C3 to C4 alkyl;
b) Di- or triester solvents of formula IV: R1(C=OOR2)n, wherein:
   R1 is a saturated or unsaturated C2 to C4 alkyl;
   R2 is independently selected from a linear or branched C2 to C8 alkyl, preferably a linear or branched C2 to C6 alkyl, most preferably a linear of branched C3 to C4 alkyl;
   n is 2 or 3 preferably 2;

Suitable monoester solvents of formula III include but are not limited to ethylacetate, propylacetate, isopropylacetate, butylacetate, isobutylacetate, amylacetate, isoamylacetate, hexylacetate, isohexylacetate, heptylacetate, isoheptylacetate, octylacetate, isooctylacetate, 2-ethylhexylacetate, ethylpropionate, propylpropionate, isopropylpropionate, butylpropionate, isobutylpropionate, amylpropionate, isoamylpropionate, hexylpropionate, isohexylpropionate, heptylpropionate, isoheptylpropionate, octylpropionate, isooctylpropionate, 2-ethylhexylpropionate, ethylbutyrate, propylbutyrate, isopropylbutyrate, butylbutyrate, isobutylbutyrate, amylbutyrate, isoamylbutyrate, hexylbutyrate, isohexylbutyrate, heptylbutyrate, isoheptylbutyrate, octylbutyrate, isooctylbutyrate, 2-ethylhexylbutyrate, ethylisobutyrate, propylisobutyrate, isopropylisobutyrate, butylisobutyrate, isobutylisobutyrate, amylisobutyrate, isoamylisobutyrate, hexylisobutyrate, isohexylisobutyrate, heptylisobutyrate, isoheptylisobutyrate, octylisobutyrate, isooctylisobutyrate, 2-ethylhexylisobutyrate, ethylpentanoate, propylpentanoate, isopropylpentanoate, butylpentanoate, isobutylpentanoate, amylpentanoate, isoamylpentanoate, hexylpentanoate, isohexylpentanoate, heptylpentanoate, isoheptylpentanoate, octylpentanoate, isooctylpentanoate, 2-ethylhexylpentanoate, ethylisopentanoate, propylisopentanoate, isopropylisopentanoate, butylisopentanoate, isobutylisopentanoate, amylisopentanoate, isoamylisopentanoate, hexylisopentanoate, isohexylisopentanoate, heptylisopentanoate, isoheptylisopentanoate, octylisopentanoate, isooctylisopentanoate, 2-ethylhexylisopentanoate, and mixtures thereof.

Preferred monoester solvents of formula III can be selected from the group consisting of ethylpropionate, propylpropionate, isopropylpropionate, butylpropionate, isobutylpropionate, amylpropionate, isoamylpropionate, hexylpropionate, isohexylpropionate, ethylbutyrate, propylbutyrate, isopropylbutyrate, butylbutyrate, isobutylbutyrate, amylbutyrate, isoamylbutyrate, hexylbutyrate, isohexylbutyrate, ethylisobutyrate, propylisobutyrate, isopropylisobutyrate, butylisobutyrate, isobutylisobutyrate, amylisobutyrate, isoamylisobutyrate, hexylisobutyrate, isohexylisobutyrate, and mixtures thereof.

Most preferably, the monoester solvents are selected from the group consisting of propylpropionate, isopropylpropionate, butylpropionate, isobutylpropionate, propylbutyrate, isopropylbutyrate, butylbutyrate, isobutylbutyrate, propylisobutyrate, isopropylisobutyrate, butylisobutyrate, isobutylisobutyrate, and mixtures thereof.

Suitable di- or tri-ester solvents of formula IV can be selected from: ethyl-, propyl-, isopropyl-, butyl-, isobutyl-, amyl-, isoamyl-,hexyl-, isohexyl-, heptyl-, isoheptyl, octyl-, isooctyl-, 2-ethylhexy- di- or tri-esters of succinic acid, glutaric acid, adipic acid, maleic acid, fumaric acid, glutaconic acid, citric acid, aconitic acid, propane-1,2,3-tricarboxylic acid, and mixtures thereof.

Preferred di- or tri-ester solvents are selected from the group consisting of ethyl-, propyl-, isopropyl-, butyl-, isobutyl-, amyl-, isoamyl-,hexyl-, isohexyl- di- or tri-esters of succinic acid, glutaric acid, adipic acid, maleic acid, fumaric acid, glutaconic acid, citric acid, aconitic acid, propane-1,2,3-tricarboxylic acid, and mixtures thereof.

More preferably, the di- or tri-ester solvents are selected from the group consisting of ethyl-, propyl-, isopropyl-, butyl-, isobutyl- di- or tri-esters of succinic acid, glutaric acid, adipic acid, maleic acid, fumaric acid, glutaconic acid, citric acid, aconitic acid, propane-1,2,3-tricarboxylic acid, and mixtures thereof.

### Alkalinity source:

The composition can comprise a source of alkalinity to reach the desired pH. Suitable sources of alkalinity for use herein are the alkali metals, such as sodium hydroxide, potassium hydroxide and/or lithium hydroxide; alkali metal carbonates such as sodium carbonate and/or potassium carbonate; alkali metal bicarbonates such as sodium bicarbonate and/or potassium bicarbonate; ethanolamines such as monoethanolamine and/or triethanolamine; and mixtures thereof.

Preferably, the source of alkalinity is selected from: sodium hydroxide, potassium hydroxide, sodium bicarbonate, potassium bicarbonate, sodium carbonate, potassium carbonate, monoethanolamine, triethanolamine, and mixtures thereof, more preferably sodium hydroxide, potassium hydroxide, sodium bicarbonate, potassium bicarbonate, sodium carbonate, potassium carbonate, monoethanolamine, and mixtures thereof. The source of alkalinity preferably comprises a bicarbonate, especially potassium bicarbonate.

Typically the amount of source of alkalinity is from 0.1% to 10% by weight, preferably from 0.25% to 5.0% and more preferably from 0.5% to 3.0% by weight of the composition, and can depend on the amount of acid used as well as the desired pH.

The source of alkalinity can comprise carbonate. The composition can comprise the carbonate at a level of from 0.1% to 5.0%, preferably from 0.25% to 4.0%, more preferably from 0.5% to 2.5% by weight of the composition.

Suitable carbonates can be selected from: sodium bicarbonate, potassium bicarbonate, sodium carbonate, potassium carbonate, and mixtures thereof, preferably sodium bicarbonate, potassium bicarbonate, and mixtures thereof, more preferably potassium bicarbonate.

Depending on the pH the bicarbonate may in situ be partially or entirely converted to carbonate.

Preferably the source of alkalinity comprises or even consists of carbonate and alkanolamine, such as monoethanolamine and/or triethanolamine, with monoethanolamine being more preferred. Alkali metal hydroxides such as sodium hydroxide can also be added, typically in small amounts, such as to adjust the pH to the desired value.

### Foam dispenser:

Foam dispensers dispense the cleaning composition as a foam. Foam dispensers combine the cleaning composition with air during dispensing, typically mixing the cleaning composition and air in a turbulent manner.

Foam dispensers include spray dispensers whereby the liquid is dispensed under pressure as droplets that are mixed with the turbulent air during dispensing to provide the foam. Alternatively, the foam dispenser can comprise a mixing chamber in which the air and the liquid composition are combined to produce the foam. The latter dispenser typically comprises a mesh and/or orifice either in the mixing chamber or after the mixing chamber through which the liquid hand dishwashing composition and air are mixed to generate the foam.

Spray dispensers comprise a reservoir to accommodate the composition of use in the invention and spraying means. The reservoir is typically a plastic container, preferably plastic bottle. To enhance the recyclability, the container can be constructed entirely from a single type of plastic, such as selected from either polyethylene or polypropylene.

The container can typically comprise from 200 ml to 5000 ml, preferably from 350 ml to 2000 ml, more preferably from 400 ml to 1000 ml of the liquid hand dishwashing detergent composition.

Suitable spray dispensers include hand pump (sometimes referred to as "trigger") devices, pressurized can devices, electrostatic spray devices, etc. Preferably the spray dispenser is non-pressurized and the spray means are of the trigger dispensing type. The reservoir is typically a container such as a bottle, more typically a plastic bottle.

The spray dispenser typically comprises a trigger lever which, once depressed, activates a small pump. The main moving element of the pump is typically a piston, housed inside a cylinder, with the piston pressing against a spring. By depressing the trigger, the piston is pushed into the cylinder and against the spring, compressing the spring, and forcing the composition contained within the pump out of a nozzle. Once the trigger lever is released, the spring pushes the piston back out, expanding the cylinder area, and sucking the composition from the reservoir, typically through a one-way valve, and refilling the pump. This pump is typically attached to a tube that draws the composition from the reservoir into the pump. The spray dispenser can comprise a further one-way valve, situated between the pump and the nozzle.

The nozzle comprises an orifice through which the composition is dispensed. The nozzle utilises the kinetic energy of the composition to break it up into droplets as it passes through the orifice. Suitable nozzles can be plain, or shaped, or comprise a swirl chamber immediately before the orifice. Such swirl chambers induce a rotary fluid motion to the composition which causes swirling of the composition in the swirl chamber. A film is discharged from the perimeter of the orifice which typically results in dispensing the composition from the orifice as finer droplets.

Since such trigger-activated spray dispensers comprise a pump, the composition preferably is not pressurized within the reservoir and preferably does not comprise a propellant.

The spray dispenser can be a pre-compression sprayer which comprises a pressurized buffer for the composition, and a pressure-activated one-way valve between the buffer and the spray nozzle. Such precompression sprayers provide a more uniform spray distribution and more uniform spray droplet size since the composition is sprayed at a more uniform pressure. Such pre-compression sprayers include the Flairosol^{®} spray dispenser, manufactured and sold by Afa Dispensing Group (The Netherlands) and the pre-compression trigger sprayers described in U.S. Patent Publication Nos. 2013/0112766 and 2012/0048959.

Alternatively, the foam dispenser can comprise a mixing chamber with at least one liquid inlet and at least one air inlet. The liquid inlet and air inlet respectively allows ingress of the cleaning composition and air into the mixing chamber, where turbulent mixing results in tehj formation of foam. A foam outlet from the mixing chamber allows dispensing of the generated foam. Foaming can be improved when the mixing chamber or the foam outlet comprise a constriction (such as an orifice) or mesh. Suitable foam dispensers are described in WO2019216272. EP Application EP25182888.5 discloses a suitable foaming bottom-dispensing package. EP Application EP25183528.6 discloses a container suitable for use with foam dispensers.

The foam dispenser may be advertised as refillable, or even sold in combination with a refill. The refill may facilitate refilling of the refillable container and may comprise a spout which facilitates the refilling thereof. In such configurations, the second container may comprise a spout that facilitates refilling of the first size container. Such spouts are described in U.S. Patent Application Publication No. 2025/0066061.

In order to facilitate recycling, the containers of the present disclosure may comprise a plastic material selected from either one of polyethylene or polypropylene. Similarly, the cap comprising the dispensing means may comprise a plastic material selected from polyethylene or polypropylene. Preferably, the plastic material of the container is substantially similar to the plastic material of the cap.

Still other containers, and especially refill containers, which may be utilized which facilitate recycling comprise film laminate materials, i.e., flexible containers. Such flexible containers may be configured as a pouch. Any suitable material may be utilized, preferable are polymeric films.

Flexible containers, particularly pouches made from polymeric films, offer a sustainable packaging solution that facilitates recycling. These containers can be single-layer or multilayer, utilizing ethylene-based polymers such as high-density polyethylene (HDPE), low-density polyethylene (LDPE), and polypropylene (PP). The inclusion of ethylene/α-olefin copolymers like ethylene-hexene in the outer layers enhances mechanical and optical properties while reducing issues like blocking.

Multilayer films are manufactured through processes such as coextrusion and laminating, with orientation improving printability and processing. Additionally, barrier layers can be added to limit oxygen and moisture transmission while allowing controlled diffusion of fragrances. Such configurations are described in additional detail in U.S. Patent Application Publication No. 2021/0229409. Non-limiting examples of barrier materials include ethylene vinyl alcohol, polyamide, and metallized films (e.g., vacuum metalized films, for example vacuum metalized polyethylene terephthalate) or inorganic oxide coatings (e.g., silica oxides, aluminum oxides). Suitable examples of multilayer films for packaging are further described in U.S. Patent Application Publication Nos. 2022/0072832 and 2018/0370202.

Sustainability can be achieved through the use of renewable feedstocks and the integration of post-consumer recycled (PCR) or post-industrial recycled (PIR) plastics, supporting a circular economy. Mono-material constructions enhance recyclability by ensuring all components are from a single polymer family, preventing contamination of recycling streams. This design approach enables optimal performance while maintaining recycling integrity.

### Method of use:

The cleaning products, as described herein, are particularly suited for methods of cleaning dishware comprising the steps of: optionally pre-wetting the dishware; applying the cleaning composition onto the dishware as a foam; optionally scrubbing the dishware; and rinsing the dishware. The composition is applied as a foam using a foaming dispenser, such as a spray dispenser.

The cleaning products described herein are particularly effective at loosening soils, and especially greasy soils. As such, especially for light soiling, scrubbing is optional, and particularly when the dishware is left for at least 15 seconds, preferably at least 30 seconds after the spray step, before the rinsing step is done.

The steps of scrubbing of the dishware and rinsing the dishware can take place at least partially simultaneously, for example, by scrubbing the dishware under running water. The scrubbing step can take between 1 second and 30 seconds.

The present method allows for faster and easier cleaning of dishware when the dishware is lightly soiled. When the dishware is heavily soiled with tough food soils such as cooked-, baked- or burnt-on soils, the present method facilitates the cleaning when the soiled dishware is soaked with the product of the invention in neat form or diluted in water, preferably for a period of from 1 second to 30 seconds, or longer.

### METHODS

### Viscosity:

The viscosity of cleaning compositions was measured at 20°C using an Automatic Kinematic Viscometer, Anton Paar SVM 2001. The autosampler aspirates the liquid and fills the measuring cell automatically. The sample is brought to the target temperature of 20°C using a Peltier element after which the measurement starts. The reported viscosity (mPa.s) is the dynamic viscosity that is measured based on a modified Couette principle according to ASTM D7042.

### pH:

The pH is measured neat (no dilution) at 20 °C, using a pH meter, such as an Orion Model 720A with an Ag/AgCl electrode (for example an Orion sure flow Electrode model 9172BN), calibrated using standardized pH 7 and pH 10 buffers.

### Reserve alkalinity:

The reserve alkalinity provides a measure of the capacity of the blend to neutralise any acid that is present, added, or formed *in-situ,* and hence maintain an alkaline pH. The reserve alkalinity for a solution is determined in the following manner:
The reserve alkalinity is measured on a 10% solution of the concentrated surfactant blend in deionized CO2-free water at 20°C. Deionized CO2-free water is deionized water that has been vigorously boiled for not less than 5 min, then cooled and protected from absorption of atmospheric carbon dioxide. 5g of the concentrated surfactant blend is diluted to 10% using 45g of deionized CO2-free water and mixed for at least 5 minutes until fully homogenized, to form a diluted blend. An aliquot of the diluted blend is weighed out and titrated using an automated titrator, such as the Omnis sample robot and Omnis titrator, supplied by Metrohm, using 0.1 N hydrochloric acid (HCl) and pH meter (as above), until an end-point of pH 7.0 is achieved or the maximum titration amount of the titrator is reached (25 ml for the titrator above). The volume of the titrant required to reach a pH of 7.0 is recorded, or if the maximum titration amount is reached, the final pH and volume of titrant is recorded.

The reserve alkalinity is calculated as %NaOH (g NaOH/g sample) equivalence of the number of millilitres, to the nearest 0.1 mL of 0.100 N hydrochloric acid (HCl) required for the titration to a pH of 7.0 of the sample.

The reserve alkalinity is calculated as %NaOH equivalence as follows: $% NaOH = \frac{\left(mL of 0.100N HCl\right) x 0.1 x 40 x 100}{\left(titrated sample weight in g\right) x 1000}$

The titrated sample weight is calculated as: $titrated sample weight in g = \frac{weight of surfactant blend \left(5g\right) \times titrated sample weight in g}{\left(diluted blend weight in g\right) \times 1000}$

Where the maximum titration amount from the titrator has been reached, the final pH is recorded and the reserve alkalinity to this pH is calculated. The reserve alkalinity to pH 7.0 can then be estimated through linear extrapolation to pH 7.0 of the obtained "pH versus volume titrant" titration curves between an added titrant volume of 20ml and 25 ml.

### Crystalline fat removal efficacy:

The crystalline greasy soil that was used for the comparative test comprised a mixture of CABF (Consumer Average Beef Fat, L2802405/200B3/E3 supplied by: J&R coordinating services Inc, Ohio, USA) and a violet dye added at a level of 0.5 wt% (Solvent Violet 13, CAS 81-48-1). The crystalline greasy soil was prepared by melting the CABF in a 60°C oven until fully liquefied, before mixing in the dye until homogeneous. The mixture was subsequently allowed to cool at ambient conditions and stored in a fridge for later use.

The dyed CABF was heated up to 60° C to liquify, before being deposited on to an enamel tile (white enamel tile, of size 25cm by 7 cm, supplied by Emaillerie Belge, Rue Saint-Denis 122, 1190 Forest, BE) homogeneously over the full surface of the tile using a synthetic foam paint roller until an amount of 0.80 ± 0.15g of the dyed CABF had been deposited onto the tile. The tile is left 3 hours at 23 °C and 50RH humidity before being used in the test.

The tile was then positioned on a rack at a 45° angle, resting on its longest side. 7ml of the test liquid composition was then pipetted dropwise via a plastic Pasteur pipette on to the tile for 1 min and the tile left for 1 further minute. The loose soil was subsequently gently washed away by pipetting between 5 and 7 ml of demineralized water dropwise for 1 min over the treated area.

After drying for 3 hours in ambient conditions (20 to 30 °C), the tile was placed horizontally and analyzed using image analysis (image captured using a Panasonic DMC-FZ45 14MP camera). A rectangular area of interest is drawn around the treated surface. The % of white pixels, a measure for grease removal, was calculated as number of white pixels / total number of pixels in area of interest x 100%

The definition for 'white pixel' was based on the following RGB values: R ≥ 150, G ≥ 180, B ≥ 150 values.

### EXAMPLES

The following cleaning compositions of use in foaming cleaning products were prepared by mixing, and the crystalline grease removal was assessed using the test method described herein. The compositions and resultant grease removal is also given in table 1.

Comparative composition A was the reference composition and did not comprise any alkanediol or aminocarboxylate chelant. Comparative example B differed from comparative example A by further comprising an alkanediol (1,2-octanediol). Comparative example C differed from comparative example A by further comprising an aminocarboxylate chelant (GLDA). Inventive example 1 differed from comparative example A by comprising both the alkanediol (1,2-octanediol) and aminocarboxylate chelant (GLDA).

**Table 1: Liquid detergent spray formulations**

| wt% (as 100% active) | Ex. A* | Ex. B* | Ex. C* | Ex. 1 |
|---|---|---|---|---|
| C8 to C10 alkyl polyglucoside¹ | 2.0 | 2.0 | 2.0 | 2.0 |
| C6 EO5 alkyl ethoxylate² | 2.0 | 2.0 | 2.0 | 2.0 |
| C12 to C14 dimethylamine oxide | 5.0 | 5.0 | 5.0 | 5.0 |
| Cocoamidopropyl betaine | 1.5 | 1.5 | 1.5 | 1.5 |
| Dipropyleneglycol n-butyl ether³ | 2.0 | 2.0 | 2.0 | 2.0 |
| 1,2-octanediol | - | 1.5 | - | 1.5 |
| GLDA⁴ | - | - | 1.0 | 1.0 |
| Monoethanolamine | 1.0 | 1.0 | 1.0 | 1.0 |
| Phenoxyethanol | 0.3 | 0.3 | 0.3 | 0.3 |
| Perfume | 0.3 | 0.3 | 0.3 | 0.3 |
| pH | 11.1 | 11.1 | 11.1 | 11.1 |
| | | | | |
| Crystalline grease removal efficacy (%) | 5% | 12% | 10% | 30% |

| | | | | |
|---|---|---|---|---|
| ¹ Glucopon^{®} 215, supplied by BASF ² Lutensol^{®} CS6250, supplied by BASF ³ Dowanol^{®} DPnB, supplied by DOW ⁴ L-glutamic acid N, N-diacetic acid sodium (GLDA) * Comparative | | | | |

From the comparative tests, it can be seen that the addition of alkanediol and aminocarboxylate chelant, individually, into compositions of use in foaming products, leads to small improvements only in crystalline grease removal without the need for excessive scrubbing (from comparing the crystalline grease removal of comparative examples B and C to comparative example A). From the result of inventive example 1, it can be seen that the combination of the alkanediol and the aminocarboxylate chelant leads to a much greater improvement in crystalline grease removal than would be expected from the alkanediol and chelants individually.

The compositions were placed into spray containers (as used for Dawn^{™} Powerwash, sold in USA), sprayed onto the prepared tiles, and the grease cleaning efficacy compared visually. The results were consistent with those from the tests of table 1.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm".

## Claims

1. A cleaning product comprising a foaming dispenser and a cleaning composition, the composition is housed in the foam dispenser and wherein the cleaning composition comprises:
a. from 2.0% to 25% by weight of the composition of a surfactant system;
b. an alkanediol, wherein the alkyl chain of the alkanediol comprises from 6 to 14 carbon atoms; and
c. an aminocarboxylate chelant.

2. The composition according to claim 1, wherein the composition comprises the alkanediol at a level of from 0.1% to 10%, preferably from 0.25% to 4.0%, more preferably from 0.25% to 3.0%, most preferably from 0.5% to 2.0% by weight of the composition.

3. The composition according to any preceding claim, wherein the alkyl chain of the alkanediol is a linear alkyl chain, preferably wherein the alkanediol is a linear 1,2-alkanediol;
preferably wherein the alkanediol is a linear 1,2-alkanediol selected from the group consisting of: 1,2-hexanediol, 1,2-octanediol, 1,2-decanediol, 1,2-dodecanediol, 1,2-tetradecanediol, or mixtures thereof, preferably 1,2-octanediol, 1,2-decanediol, 1,2-dodecanediol, and mixtures thereof, most preferably 1,2-octanediol, 1,2-decanediol, and mixtures thereof.

4. The composition according to any preceding claim, wherein at least 50%, preferably at least 80%, more preferably at least 98% by weight of the 1,2-alkanediols present in the composition are C8 to C12 1,2-alkanediols, preferably wherein at least 50%, preferably at least 80%, more preferably at least 98% by weight of the 1,2-alkanediols present in the composition is 1,2-octanediol.

5. The composition according to any preceding claim, wherein the aminocarboxylate chelant is selected from: methyl glycine diacetate (MGDA), L-glutamic acid N, N-diacetic acid sodium (GLDA), ethylenediamine-N,N'-disuccinic acid (EDDS), and mixtures thereof, preferably methyl glycine diacetate (MGDA), L-glutamic acid N, N-diacetic acid sodium (GLDA), and mixtures thereof, more preferably L-glutamic acid N, N-diacetic acid sodium (GLDA).

6. The composition according to any preceding claim, wherein the aminocarboxylate chelant is present at a level of from 0.1% to 10%, preferably from 0.2% to 5%, more preferably from 0.2% to 3%, most preferably from 0.5% to 1.5% by weight of the composition.

7. The cleaning product according to any preceding claim, wherein the cleaning composition comprises from 5.0% to 20%, preferably from 7.5% to 15% by weight of the composition of a surfactant system.

8. The cleaning product according to any preceding claim, wherein the surfactant system comprises a first surfactant selected from the group consisting of: amphoteric surfactant, zwitterionic surfactant, and mixtures thereof, preferably wherein the first surfactant is present at a level of at least 25%, preferably from 40% to 90%, more preferably from 50% to 75% by weight of the surfactant system.

9. The cleaning product according to claim 8, wherein the first surfactant comprises, preferably consists of amphoteric surfactant, wherein the amphoteric surfactant is selected from amine oxide surfactant, preferably wherein the amine oxide surfactant is selected from the group consisting of: alkyl dimethyl amine oxide, alkyl amido propyl dimethyl amine oxide, and mixtures thereof, more preferably alkyl dimethyl amine oxide.

10. The cleaning product according to claim 8, wherein the first surfactant comprises, preferably consists of zwitterionic surfactant, wherein the zwitterionic surfactant is a betaine surfactant, preferably wherein the betaine surfactant is selected from the group consisting of: cocamidopropyl betaine, cocobetaines, lauramidopropyl betaine, lauryl betaine, myristyl amidopropyl betaine, myristyl betaine, and mixtures thereof, preferably cocamidopropyl betaine.

11. The cleaning product according to any preceding claim, wherein the surfactant system further comprises alkyl polyglucoside nonionic surfactant, preferably wherein the surfactant system comprises the alkyl polyglucoside nonionic surfactant at a level of from 0.5% to 7.5%, more preferably from 0.75% to 5.0%, most preferably from 1.0% to 3.5% by weight of the composition;
preferably wherein the alkyl polyglucoside comprises or consists of short-chain alkyl polyglucoside nonionic surfactant, wherein the short-chain alkyl polyglucoside nonionic surfactant has an average alkyl carbon chain length of from 8 carbon atoms to 10 carbon atoms and a number average degree of polymerization of from 0.5 to 3.0.

12. The cleaning product according to any preceding claim, wherein the surfactant system further comprises alkoxylated nonionic surfactant, preferably wherein the surfactant system comprises the alkoxylated nonionic surfactant at a level of from 0.5% to 10%, preferably from 1.0% to 5.0%, more preferably from 1.5% to 3.0% by weight of the composition;
preferably wherein the alkoxylated nonionic surfactant comprises or consists of a low-cut alkyl ethoxylate nonionic surfactant, wherein the low-cut alcohol ethoxylate nonionic surfactant has an average alkyl carbon chain length of C10 and below, preferably from C5 to C8, more preferably C5 to C7; and a number average degree of ethoxylation of from 1.0 to 10, preferably from 3.0 to 8.0, more preferably from 4.0 to 6.0.

13. The composition according to any preceding claim, wherein the surfactant system comprises less than 25% by weight of the surfactant system of anionic surfactant, preferably less than 15%, preferably less than 10%, more preferably less than 5% by weight of the surfactant system, and most preferably wherein the surfactant system is essentially free, or even free of anionic surfactant.

14. The composition according to any preceding claim, wherein the composition further comprises glycol ether solvent, preferably wherein the glycol ether solvent is selected from the group consisting of: alkyl ethers of ethylene glycol or propylene glycol, and mixtures thereof.

15. The composition according to any preceding claim, wherein the composition comprises a source of alkalinity, wherein the source of alkalinity is selected from: sodium hydroxide, potassium hydroxide, sodium bicarbonate, potassium bicarbonate, sodium carbonate, potassium carbonate, monoethanolamine, triethanolamine and mixtures thereof, preferably sodium hydroxide, potassium hydroxide, sodium bicarbonate, potassium bicarbonate, sodium carbonate, potassium carbonate, monoethanolamine, and mixtures thereof, more preferably wherein the source of alkalinity comprises a bicarbonate.
